# EUROPEAN PATENT APPLICATION

(11) **EP 4 570 260 A1**
(43) Date of publication of application: **18.06.2025**
(21) Application number: 23216890.6
(22) Date of filing: 14.12.2023
(51) Int. Cl.: A61K 35/76, A61K 48/00, C07K 14/005, C12N 5/10, C12N 15/86

(54) **VIRAL VECTOR FOR TRANSDUCTION OF ADIPOCYTES**

(71) Applicant: Medizinische Hochschule Hannover, 30625 Hannover (DE); Syddansk Universitet, 5230 Odense M (DK)
(72) Inventor: Büning, Prof. Dr., Hildegard, Hannover (DE); Kornfeld, Prof. Dr., Jan-Wilhelm, Odense (DK); Jäschke, Dr., nico, Hannover (DE); Ruppert, Dr., Philip, Odense (DK); Pradas-Juni, Dr., Marta, Odense (DK); Güller, Aylin Seren, Odense (DK)
(74) Representative: Taruttis, Stefan Georg

(57) **Abstract**

Viral vectors based on adeno-associated virus (AAV), preferably AAV serotype 1 (AAV1), are provided for use in transduction of adipocytes. The viral vectors of the invention have the advantage of being adapted in tropism for adipocytes, wherein the viral vectors especially in use for intraperitoneal administration are optimized for adipocytes residing in SCAT (subcutaneous adipose tissue) or in VAT (visceral adipose tissue), or the viral vectors in use for oral administration are optimized for adipocytes residing in SCAT or in VAT.

## Description

The present invention relates to viral vectors based on adeno-associated virus (AAV), preferably AAV serotype 1 (AAV1), for use in transduction of adipocytes, especially for use in *in vivo* or in *ex vivo* transduction of adipocytes, especially for genetic manipulation of adipocytes. The viral vectors of the invention have the advantage of being adapted in tropism for adipocytes, wherein the viral vectors especially in use for intraperitoneal administration are optimized for adipocytes residing in SCAT (subcutaneous adipose tissue, also termed subcutaneous white adipose), or the viral vectors in use for intraperitoneal administration are optimized for adipocytes residing in VAT (visceral adipose tissue, also termed visceral white adipose), or the viral vectors in use for oral administration are optimized for adipocytes residing in SCAT, or the viral vectors in use for oral administration are optimized for adipocytes residing in VAT.

Accordingly, the viral vectors of the invention are engineered for targeting and transducing adipocytes of SCAT or adipocytes of VAT, in each case either by intraperitoneal administration or by oral administration.

The viral vectors of the invention have the advantage of being optimized for transducing adipocytes with specificity for SCAT or VAT, which specificity is also dependent on and can be controlled by the route of administration, and therefore are suitable for use in medical treatment by expressing a nucleic acid construct in adipocytes of SCAT or VAT. Adipocytes and adipose tissue as a target of the viral vectors of the invention have the advantage that no tumours are known that originate from adipose tissue, no fibrogenesis is known to originate from adipose tissue, generally, adipose tissue can be removed without an organ loosing its function, and in comparison to e.g. liver immune responses against adipose tissue are generally low due to low presence of antigen presenting cells in adipose tissue, and genetic manipulation of adipose tissue is expected to be long-lived, e.g. stable, as adipocytes are non-proliferating and long-lived.

### State of the art

Bates et al., Molecular Therapy: Methods & Clinical Development Vol. 19, 236-249 (2020) review AAV-based vectors for targeting adipose tissue, quoting low transduction efficiency for AAV1 even when directly injected into adipose tissue.

Hacker et al., The Journal of Gene Medicine 2005; 7:1429-1438 describes a self-complementary AAV vector genome configuration.

### Object of the invention

It is an object of the invention to provide viral vectors having tropism for adipocytes, suitable for transducing adipocytes, wherein preferably the viral vectors have specificity for SCAT or VAT. More preferred, the viral vectors shall have optimized transduction abilities for SCAT or VAT, depending on the route of administration, e.g. depending on oral or intraperitoneal administration.

### Description of the invention

The invention achieves the object by the features of the claims and especially provides an AAV vector, preferably an AAV1 vector, which in the VP1 reading frame of the capsid ORF (capsid protein) C-terminally to amino acid No. 590, which is aspartate (D), contains an insert comprising one of SEQ ID NO: 1 to SEQ ID NO: 80, wherein preferably the insert is flanked by 2 to 3 amino acid residues as a linker, preferably alanine residues, e.g. 3 alanine residues between amino acid No. 590 and the insert of one of SEQ ID NO: 1 to SEQ ID NO: 80, and C-terminally to the insert contains 2 alanine residues as a linker, with the remainder of the VP1 reading frame arranged C-terminally to these alanine residues. The insert, preferably flanked both N-terminally and C-terminally by a linker of 2 to 3 amino acids as a linker, e.g. 2 to 3 alanine residues, is arranged between amino acid No. 590 and amino acid No. 591 of the wild-type sequence of VP1 (virion protein 1, SEQ ID NO: 81) reading frame of the capsid ORF, e.g. of AAV1. The capsid of the invention, which contains one of the inserts, which insert is flanked by an N-terminal linker of 3 alanine residues (amino acids 591..593) and a C-terminal linker of 2 alanine residues (amino acids 601..602) is represented by SEQ ID NO: 82, wherein the insert is represented by a section of seven wildcard amino acids (No. 594..600). Accordingly herein, each of the amino acid sequences of SEQ ID NO: 1 to SEQ ID NO: 80 can replace the section of the seven wildcard amino acids of SEQ ID NO: 82. The viral vector of the invention is suitable for use in medical treatment of adipocytes, especially for transduction adipocytes, e.g. transducing adipocytes with a nucleic acid construct, which e.g. contains a nucleic acid section for recombination with chromosomal DNA of an adipocyte, a nucleic acid encoding a protein, and/or a nucleic acid encoding an RNA, e.g. encoding an inhibitory RNA, an mRNA, a long noncoding RNA (lncRNA) or a microRNA (miRNA), or other small RNAs for use in transcriptional gain-of-function or loss-of-function methods, e.g. single guide RNA (sgRNA), small interfering RNA (siRNA), short hairpin RNA (shRNA) or other small RNAs using in transcriptional gain- or loss-of-function approaches (single guide RNA, small interfering RNA, short hairpin RNA), or for use of the AAV vector for the transfer of genome editing compounds and/or nucleic acid sequences as templates for use of the homology-directed repair (HDR).

Generally, the process for producing AAV-based viral vector particles according to the invention can be by delivery, e.g. by plasmid transfection with or without helper virus co-infection, of all required components for AAV vector production, e.g. from a vector genome containing the transgene expression cassette flanked by ITRs or a nucleic acid sequence for HDR, AAV rep and AAV cap genes as well as other viral helper genes necessary for AAV particle production, e.g. from adenovirus. The process can be performed in a cultivated eukaryotic host cell, followed by cell lysis and removal of cellular components and plasmid DNA, e.g. by enzymatic digestion, filtration and/or centrifugation, and further purification, e.g. by gradient density centrifugation and/or chromatography of AAV viral vector particles. In a specific embodiment, empty viral particles comprising the protein of the VP1 reading frame of the capsid ORF (capsid protein) with the insert according to the invention are provided, which do not contain a nucleic acid molecule. These empty viral particles can e.g. be associated with a functional molecule for delivery of the functional molecule to adipocyte tissue, preferably specifically to SCAT or VAT. The functional molecule can e.g. be a therapeutic agent or an indicator compound, e.g. a dye or a pharmaceutically acceptable diagnostic contrast agent, or a combination of at least two of these. Empty viral particles can be produced e.g. in HEK293 cells. The process comprises the steps of transfecting the respective helper plasmid (containing the *cap* gene, if applicable with an inserted amino acid section, and the *rep* gene of wild-type AAV1) and an adenoviral helper plasmid (containing adenoviral helper functions required for AAV vector production) in HEK293 cells for AAV empty capsid production (no vector genome plasmid containing ITRs flanking the expression cassette is used in this specific case), with subsequent purification of empty capsid particles by iodixanol gradient centrifugation.

Preferably, the AAV vector, preferably the AAV1 vector, for use in intraperitoneal administration for transduction of adipocytes residing in SCAT contains an insert of one of SEQ ID NO: 1 to SEQ ID NO: 20, preferably SEQ ID NO: 1, SEQ ID NO: 2, or SEQ ID NO: 3;
the AAV vector, preferably the AAV1 vector, for use in intraperitoneal administration for transduction of adipocytes residing in VAT contains an insert of one of SEQ ID NO: 21 to SEQ ID NO: 40, preferably SEQ ID NO: 21, SEQ ID NO: 22, or SEQ ID NO: 23;
the AAV vector, preferably the AAV1 vector, for use in oral administration for transduction of adipocytes residing in SCAT contains an insert of one of SEQ ID NO: 41 to SEQ ID NO: 60, preferably SEQ ID NO: 41, SEQ ID NO: 42, or SEQ ID NO: 43; and
the AAV vector, preferably the AAV1 vector, for use in oral administration for transduction of adipocytes residing in VAT contains an insert of one of SEQ ID NO: 61 to SEQ ID NO: 80, preferably SEQ ID NO: 61, SEQ ID NO: 62, or SEQ ID NO: 63.

Most preferred, the AAV vector, preferably the AAV1 vector, contains one of SEQ ID NO: 61 (also termed VP48), SEQ ID NO: 62 (also termed VP51), SEQ ID NO: 41 (also termed VP50), SEQ ID NO: 42 (also termed VP49), and SEQ ID NO: 63 (also termed VP47), each for use in oral administration.

The invention is now described in greater detail by way of examples and with reference to the figures, which show in
- Fig. 1 relative transduction levels of murine adipocytes by viral vectors of the invention, indicated by expression levels of GFP, and in
- Fig. 2 relative transduction levels of human adipocytes by viral vectors of the invention, indicated by expression levels of GFP.

### Example 1: Selection of AAV1 viral particles with tropism for adipocytes from an AAV1 peptide display library

A library of AAV1 viral particles containing more than 10⁶ different inserts of 7 random amino acids arranged in the VP1 capsid protein was constructed. In short, the coding sequence for the wild-type capsid protein of SEQ ID NO: 81, containing a linker of three alanine residues - seven random amino acids - a linker of two alanine residues between amino acids No. 590 and 591 of the wild-type capsid proteins, corresponding to amino acids 590 and 603 of SEQ ID NO: 82, with the seven random amino acids comprising more than 10⁶ different inserts was constructed. This library was cloned as plasmid library by inserting oligonucleotides of random sequence flanked by sequences coding for a linker of three and two alanine residues, respectively, into the *cap* gene at a position which is between the amino acids corresponding to the amino acid positions 590 and 591 of the wild-type capsid. The plasmid library was used to produce the viral AAV peptide display library in HEK293 cells. Following an initial production step, the resultant capsid engineered viral particles were subjected to a phenotype-genotype coupling step to enable identification of the capsid modification through sequencing of the *cap* gene delivered by the AAV particles to the target cells.

The viral particles were produced in HEK producer cells. In short, the variants were produced as viral vectors in HEK293 cells using an adenoviral helper plasmid (e.g. pXX6), a vector plasmid encoding for eGFP in a self-complementary vector genome configuration and the respective helper plasmid encoding for AAV2 rep and the modified AAV1 cap gene. For each variant a unique AAV helper plasmid was used. Following vector production, cells here harvested and lysed. Viral vector particles were purified by density gradient centrifugation followed by buffer exchange.

Genomic particle titer was determined by qPCR. Subsequently, the library was subjected towards a phenotype-genotype coupling step. For this, HEK293 were seeded on top of viral particles bound to a cell culture plate by anti-AAV1 antibody. This step enables uptake of AAV particles independent of a specific targeting receptor. Due to performing this coupling step at a particle-per-cell ratio of approximately 1000, only limited number of viral genomes are entering the cell nucleus ensuring formation of uniform capsids and packaging of the cognate viral genome. Following production, the library is again subjected to a density gradient centrifugation and determination of genomic particle titers by qPCR. The titer of the library was 7×10⁹ per microliter.

Viral particles containing the different inserts were administered to C57BL/6N mice, once by intraperitoneal injection of 3.5 × 10¹⁰ viral particles per mouse suspended in 1 × PBS (intraperitoneal administration), once by oral administration of 3.5 × 10¹⁰ viral particles diluted in 20% sucrose solution.

Ten days subsequent to the intraperitoneal or oral administration, mice were sacrificed and subcutaneous white adipose tissue (SCAT) and visceral white adipose tissue (VAT) were isolated. The isolated SCAT and VAT were used to isolate mature (buoyant) floating adipocytes. From these purified adipocytes, total genomic DNA was isolated, followed by next generation sequencing (NGS) in order to identify the sequences encoding the insert introduced into each of the capsid subunits at VP1 C-terminally to amino acid 590, which insert mediated infection of adiopocytes.

In total, in SCAT for intraperitoneal administration, 38715 sequences were analysed, identifying a total of 4173 different insert sequences, in VAT for intraperitoneal administration, 53039 sequences were analysed, identifying a total of 9173 different insert sequences, in SCAT for oral administration, 15189 sequences were analysed, identifying a total of 324 different insert sequences, in VAT for oral administration, 27429 sequences were analysed, identifying a total of 470 different insert sequences. It was found that viral particles with VP1 capsid proteins containing specific inserts were enriched in either SCAT or VAT in dependence on the route of administration.

VP1 capsid proteins containing the insert sequences were ranked according to their proportion, indicating their specificity for SCAT or VAT:

| SCAT, intraperitoneal | | VAT, intraperitoneal | | SCAT, oral | | VAT, oral | |
|---|---|---|---|---|---|---|---|
| rank | SEQ ID NO: | rank | SEQ ID NO: | rank | SEQ ID NO: | rank | SEQ ID NO: |
| 2.73 | 1 | 1.15 | 21 | 25.28 | 41 | 28.54 | 61 |
| 1.36 | 2 | 1.08 | 22 | 9.92 | 42 | 15.35 | 62 |
| 0.55 | 3 | 0.51 | 23 | 9.69 | 43 | 10.67 | 63 |
| 0.55 | 4 | 0.38 | 24 | 6.15 | 44 | 8.58 | 64 |
| 0.49 | 5 | 0.32 | 25 | 6.12 | 45 | 5.22 | 65 |
| 0.40 | 6 | 0.25 | 26 | 5. 57 | 46 | 3.94 | 66 |
| 0.35 | 7 | 0.24 | 27 | 4.89 | 47 | 3.87 | 67 |
| 0.35 | 8 | 0.21 | 28 | 4.17 | 48 | 3.03 | 68 |
| 0.34 | 9 | 0.20 | 29 | 3.43 | 49 | 2.59 | 69 |
| 0.33 | 10 | 0.20 | 30 | 3.18 | 50 | 2.21 | 70 |
| 0.32 | 11 | 0.20 | 31 | 2.68 | 51 | 1.40 | 71 |
| 0.30 | 12 | 0.19 | 32 | 2.51 | 52 | 1.21 | 72 |
| 0.29 | 13 | 0.19 | 33 | 2.33 | 53 | 1.06 | 73 |
| 0.29 | 14 | 0.18 | 34 | 2.22 | 54 | 0.98 | 74 |
| 0.29 | 15 | 0.18 | 35 | 1.74 | 55 | 0.87 | 75 |
| 0.28 | 16 | 0.17 | 36 | 1.55 | 56 | 0.82 | 76 |
| 0.28 | 17 | 0.16 | 37 | 1.22 | 57 | 0.81 | 77 |
| 0.27. | 18 | 0.16 | 38 | 1.18 | 58 | 0.78 | 78 |
| 0.27 | 19 | 0.15 | 39 | 0.90 | 59 | 0.75 | 79 |
| 0.27 | 20 | 0.15 | 40 | 0.52 | 60 | 0.55 | 80 |

This result shows that from the high number of VP1 capsid proteins having different inserts that were administered, after the *in vivo* selection, the capsid proteins containing one of these inserts show tropism for adipose tissue. Interestingly, the route of administration had a strong influence on the tropism for VAT or SCAT. Accordingly, for *in vivo* use, an AAV1 vector particle having a VP1 containing an insert of one of SEQ ID NO: 1 to SEQ ID NO: 20 is preferred for intraperitoneal administration for transducing SCAT, an AAV1 vector particle having a VP1 containing an insert of one of SEQ ID NO: 21 to SEQ ID NO: 40 is preferred for intraperitoneal administration for transducing VAT, an AAV1 vector particle having a VP1 containing an insert of one of SEQ ID NO: 41 to SEQ ID NO: 60 is preferred for oral administration for transducing SCAT, and an AAV1 vector particle having a VP1 containing an insert of one of SEQ ID NO: 61 to SEQ ID NO: 80 is preferred for oral administration for transducing VAT, wherein preferably the insert is contained in a VP1 according to SEQ ID NO: 82, therein replacing the wildcard amino acid residues.

### Example 2: Transduction of adipocytes by AAV1 viral vector particles and capsid modified AAV1 derived viral vector particles

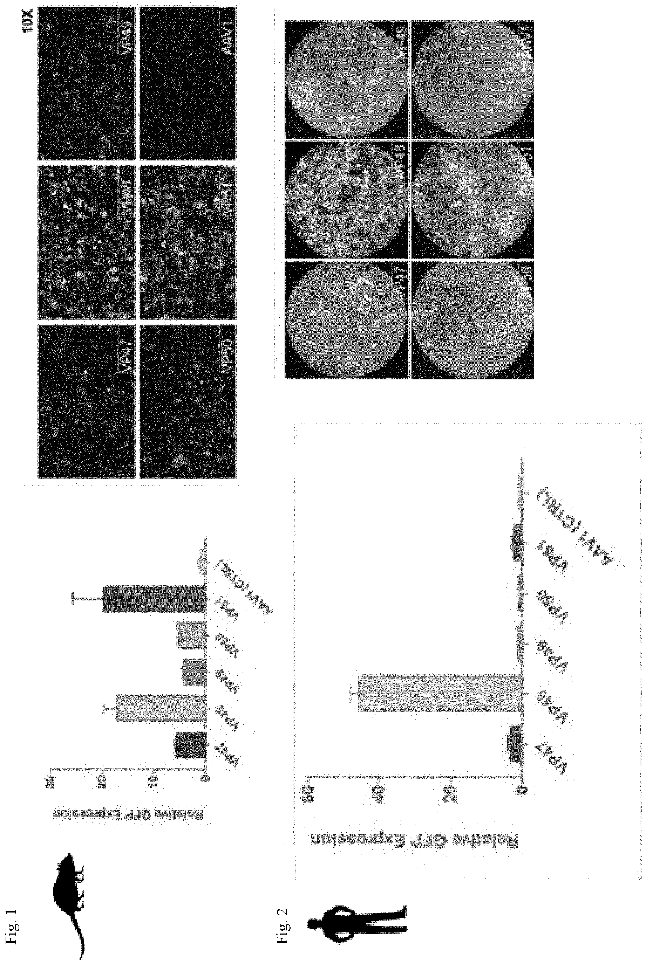

As representatives of the AAV vector particles, which preferably are AAV1 viral vector particles, of the invention, viral vector particles having the VP1 protein according to SEQ ID NO: 82 with an insert of one of SEQ ID NO: 63 (VP47), SEQ ID NO: 61 (VP48), SEQ ID NO: 42 (VP49), SEQ ID NO: 41 (VP50), and SEQ ID NO: 62 (VP51) replacing the seven wild-card amino acids, were used to transduce *in vitro* cultivated adipocytes. As a representative and indicator for successful transduction, the viral particles contained an expression cassette for enhanced GFP (eGFP) in a self-complementary AAV vector genome configuration (as described by Hacker et al., The Journal of Gene Medicine 2005; 7:1429-1438). In short, murine or human adipocytes were cultivated in standard DMEM medium to confluence, after which adipogenesis was induced by adding 1µM rosiglitazone, 850nM insulin, 1µM dexamethasone, and 250µM IBMX for a duration of eleven days. After successful adipocyte differentiation, mature adipocytes were treated with 50,000 (designation are indicated in Fig. 1) viral particles per cell for transduction to the cell culture and cells were harvested for immunofluorescence measurements and/or quantitative RT-PCR after 48h.

Fig. 1 shows expression of eGFP measured 48 h after transduction in murine adipocytes by qPCR using transgene specific primers and following normalization to levels of b-actin mRNA. Murine adipocytes were incubated with indicated vectors at a particle-per-cell ratio of 50 000. Cells were lysed and mRNA was isolated 48 hrs post vector administration. qPCR analysis was performed on cDNA of samples and shown are results normalized to housekeeping gene b-actin. Specifically, these results reveal that the preferred insert of VP51 and insert VP48, and to a lower extent the insert VP47 and VP49 efficiently transduced murine adipocytes for expression of a transgene encoded by a nucleic acid construct contained in an AAV1 particle, whereas the wild-type capsid (CTRL; AAV1) in comparison shows only background transduction activity.

Fig. 2 shows expression of eGFP measured in human adipocytes by qPCR using transgene specific primers and following normalization to levels of b-actin mRNA. Murine adipocytes were incubated with indicated vectors at a particle-per-cell ratio of 50 000. These results reveal that the preferred insert of VP48, and to a lower extent the insert VP47, VP49, VP50 and VP51 efficiently transduce human adipocytes for expression of a transgene encoded by a nucleic acid construct contained in an AAV1 particle, whereas the wild-type capsid (CTRL; AAV1) in comparison shows only background transfection activity.

## Claims

1. Viral vector for use in transducing adipocytes residing in adipose tissue, wherein the viral vector contains VP1 of AAV1 and in its VP1 between amino acid 590 and amino acid 591 of its wild-type sequence contains an insert of one of SEQ ID NO: 1 to SEQ ID NO: 80.

2. Viral vector according to claim 1 for use in transducing adipocytes residing in adipose tissue by oral administration, wherein the viral vector in its VP1 between amino acid 590 and amino acid 591 of its wild-type sequence contains an insert of one of SEQ ID NO: 41 to SEQ ID NO: 80.

3. Viral vector according to claim 1 for use in transfecting adipocytes residing in adipose tissue by intraperitoneal administration, wherein the viral vector in its VP1 between amino acid 590 and amino acid 591 of its wild-type sequence contains an insert of one of SEQ ID NO: 1 to SEQ ID NO: 40.

4. Viral vector according to one of the preceding claims, wherein the viral vector comprises a VP1 having an amino acid sequence of SEQ ID NO: 82, wherein the amino acids No. 594 to 600 are selected from SEQ ID NO: 1 to SEQ ID NO: 80.

5. Viral vector according to one of the preceding claims for use in medical treatment, wherein the viral vector contains a nucleic acid construct encoding an siRNA, a therapeutically active nucleic acid, antibodies, signal molecules, messenger molecules, hormones, genome/epigenome editing tools or providing a template for genome editing.

6. Viral vector according to one of the preceding claims for use in the treatment of a genetic defect, cancer or infectious diseases, wherein the viral vector contains a nucleic acid construct containing an expression cassette encoding a protein substituting a genetic defect.

7. Viral vector according to one of the preceding claims, wherein the viral vector is adeno-associated virus (AAV).

8. Viral vector according to one of the preceding claims, wherein the viral vector is AAV serotype 1 (AAV1).
